# EUROPEAN PATENT APPLICATION

(11) **EP 4 332 543 A1**
(43) Date of publication of application: **06.03.2024**
(21) Application number: 22795742.0
(22) Date of filing: 25.04.2022
(51) Int. Cl.: G01N 1/10, A61B 34/37, B25J 3/00

(54) **ROBOT SYSTEM**

(30) Priority: 26.04.2021 JP 2021074373
(71) Applicant: KAWASAKI JUKOGYO KABUSHIKI KAISHA, Kobe-shi, Hyogo 650-8670 (JP)
(72) Inventor: KOKUSHI, Hiroki, Hyogo 650-8670 (JP); RYU, Hideyuki, Hyogo 650-8670 (JP)
(74) Representative: Hoffmann Eitle
(86) International application number: PCT/JP2022/018784
(87) International publication number: WO 2022/230829

(57) **Abstract**

A robot system (100) includes a slave robot (10) including a hand (12) to hold a treatment member (101) and operable to perform a procedure on a person to be treated (S) using the treatment member held by the hand, a master robot (20) to remotely control the slave robot, and a controller (50) configured or programmed to restrict movement of the hand according to a step of the procedure on the person to be treated using the treatment member held by the hand.

## Description

### Technical Field

The present disclosure relates to a robot system, and more particularly, it relates to a robot system that performs a procedure such as collecting a specimen from a person to be treated.

### Background Art

Conventionally, a specimen collection box for collecting a specimen from a person to be treated is known. Such a specimen collection box is disclosed in Japanese Utility Model Registration No. 3228999, for example.

Japanese Utility Model Registration No. 3228999 discloses a specimen collection box for collecting a specimen from a person to be treated as a countermeasure against infectious diseases caused by new coronavirus (COVID-19). This specimen collection box includes a foldable box body including four side members, and a pair of protective gloves for specimen collection. This specimen collection box allows a collecting person in the box body to wear the pair of protective gloves and perform specimen collection work to collect a specimen from the person to be treated.

### Prior Art

### Patent Document

Patent Document 1: Japanese Utility Model Registration No. 3228999

### Summary of the Invention

Although not clearly described in Japanese Utility Model Registration No. 3228999, it is conceivable to use a master-slave robot system including a master robot and a slave robot to perform specimen collection work as described in Japanese Utility Model Registration No. 3228999. In this case, it is conceivable that a hand of the slave robot holds a specimen collection member and is remotely controlled by the master robot to perform the specimen collection work. However, the hand of the slave robot is relatively freely operable, and thus when the hand of the slave robot is remotely controlled by the master robot to perform the specimen collection work, unintended and unnecessary movement of the hand may disadvantageously occur. For example, when the posture of the hand is changed in order to insert the specimen collection member into a subject, unintended and unnecessary movement of the hand in a forward-rearward direction may occur. In such a case, it is difficult to easily perform the specimen collection work. A similar problem also arises when a procedure other than the specimen collection work is performed on the subject.

The present disclosure is intended to solve the above problem. The present disclosure aims to provide a robot system that enables a procedure to be easily performed on a person to be treated even when a hand of a slave robot is remotely controlled by a master robot to perform the procedure on the person to be treated.

In order to attain the aforementioned object, a robot system according to an aspect of the present disclosure includes a slave robot including a hand to hold a treatment member and operable to perform a procedure on a person to be treated using the treatment member held by the hand, a master robot to remotely control the slave robot, and a controller configured or programmed to restrict movement of the hand according to a step of the procedure on the person to be treated using the treatment member held by the hand. The term "procedure" indicates a broader concept including collecting a specimen from the person to be treated, performing an examination on the person to be treated, performing surgery on the person to be treated, etc.

As described above, the robot system according to this aspect of the present disclosure includes the controller configured or programmed to restrict movement of the hand according to the step of the procedure on the person to be treated using the treatment member held by the hand. Accordingly, movement of the hand is appropriately restricted according to the step of the procedure, and thus unlike a case in which no restriction is set on movement of the hand, unintended and unnecessary movement of the hand can be reduced or prevented. Consequently, movement of the hand suitable for the step of the procedure can be performed, and thus even when the master robot remotely controls the hand of the slave robot to perform the procedure on the person to be treated, the procedure on the person to be treated can be easily performed. Thus, the workability of the procedure on the person to be treated can be improved.

According to the present disclosure, as described above, it is possible to easily perform the procedure on the person to be treated even when the hand of the slave robot is remotely controlled by the master robot to perform the procedure on the person to be treated.

### Brief Description of the Drawings

FIG. 1 is a diagram showing a specimen collection robot system according to a first embodiment.
FIG. 2 is a diagram showing a slave robot according to the first embodiment.
FIG. 3 is a diagram showing the slave robot and a subject according to the first embodiment.
FIG. 4 is a diagram for illustrating specimen collection by a hand according to the first embodiment.
FIG. 5 is a diagram for illustrating movement of the hand according to the first embodiment.
FIG. 6 is a diagram showing a master robot according to the first embodiment.
FIG. 7 is a diagram for illustrating control of a controller according to the first embodiment.
FIG. 8 is a diagram for illustrating a step of placing a specimen collection member in front of the nasal cavity in specimen collection work according to the first embodiment.
FIG. 9 is a diagram for illustrating a step of inserting the specimen collection member into the nasal cavity in the specimen collection work according to the first embodiment.
FIG. 10 is a diagram for illustrating a step when the specimen collection member has been inserted into the nasal cavity in the specimen collection work according to the first embodiment.
FIG. 11 is a diagram for illustrating a step when the specimen collection member has reached the nasopharynx in the specimen collection work according to the first embodiment.
FIG. 12 is a flowchart for illustrating a control process for the specimen collection work according to the first embodiment.
FIG. 13 is a diagram showing a robot system according to a second embodiment.
FIG. 14 is a diagram for illustrating ureteroscopic surgery performed by operating a slave robot according to the second embodiment.
FIG. 15 is a diagram for illustrating movement of a hand according to the second embodiment.
FIG. 16 is a diagram for illustrating a step of inserting a treatment member into the urethra in the ureteroscopic surgery according to the second embodiment.
FIG. 17 is a diagram for illustrating a step of identifying a calculus site in the ureteroscopic surgery according to the second embodiment.
FIG. 18 is a diagram for illustrating a step during use of a forceps in the ureteroscopic surgery according to the second embodiment.

### Modes for Carrying Out the Invention

Embodiments embodying the present disclosure are hereinafter described on the basis of the drawings.

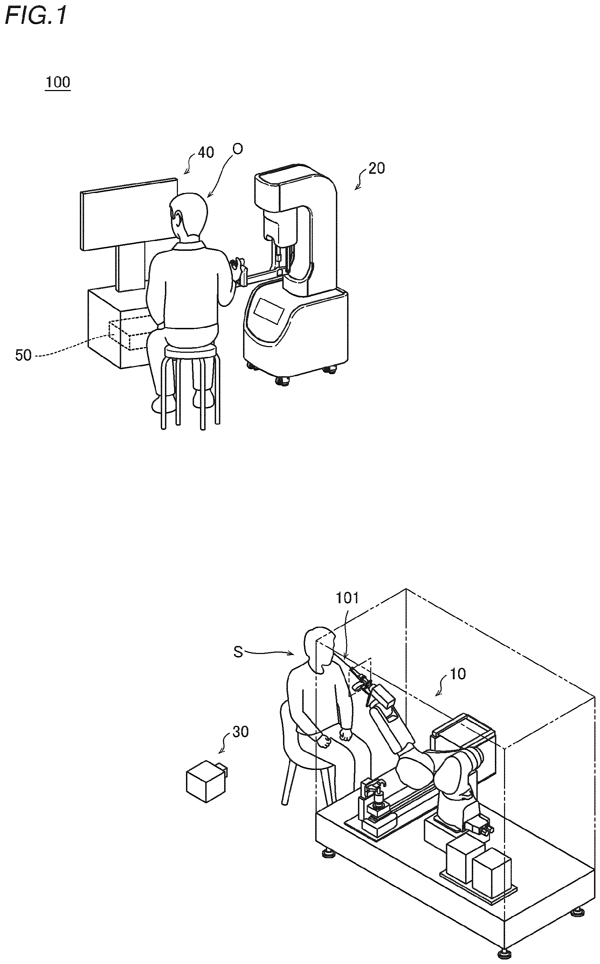

The configuration of a specimen collection robot system 100 according to a first embodiment is now described with reference to FIGS. 1 to 11. The specimen collection robot system 100 is an example of a "robot system" in the claims.

As shown in FIG. 1, the specimen collection robot system 100 includes a slave robot 10, a master robot 20, an imager 30, a display 40, and a controller 50.

As shown in FIGS. 1 to 4, the slave robot 10 collects a specimen from a subject S using a specimen collection member 101. The specimen collection member 101 is a sterile swab, for example. The sterile swab has a stick shape. The slave robot 10 inserts the specimen collection member 101 into the nasal cavity of the subject S, for example, and collects the specimen (nasopharyngeal swab) from the nasopharynx of the subject S by the inserted specimen collection member 101. The slave robot 10 may insert the specimen collection member 101 into the oral cavity of the subject S to collect the specimen. A virus test (a test for new coronavirus, for example) such as a PCR (polymerase chain reaction) test is performed on the collected specimen. In the specimen collection robot system 100, a person in charge of specimen collection, such as a doctor, does not need to face the subject S to collect the specimen, and thus it is possible to isolate the person in charge of specimen collection from the risk of infection. The specimen collection member 101 is an example of a "treatment member" in the claims. The subject S is an example of a "person to be treated" in the claims.

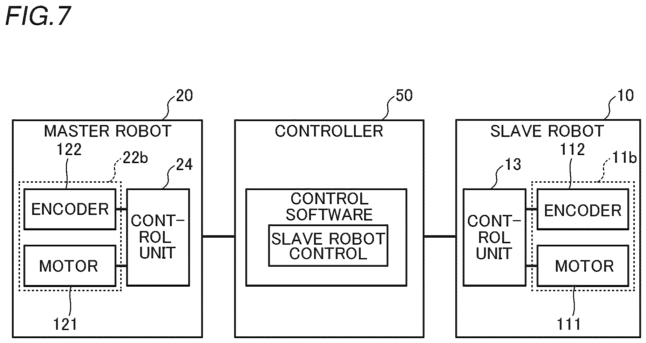

The slave robot 10 is a vertical articulated robot. The slave robot 10 includes an arm 11 and a hand 12 attached to a tip end of the arm 11. The arm 11 includes a plurality of links 11a. The plurality of links 11a are connected to each other by joints 11b. A plurality of joints 11b are provided. Each of the plurality of joints 11b includes a motor 111 (servomotor) (see FIG. 7) as a drive and an encoder 112 (see FIG. 7) as a position detector. The hand 12 holds the specimen collection member 101. The hand 12 grips and holds the specimen collection member 101 with a chuck mechanism, for example.

The slave robot 10 is arranged in a system housing 60. A control unit 13 that controls the slave robot 10 is provided inside the system housing 60.

A shielding plate 61 is arranged between the slave robot 10 and the subject S. The shielding plate 61 is installed on the system housing 60 that surrounds the slave robot 10. The shielding plate 61 is made of a colorless and transparent member such as a glass plate or an acrylic plate, and includes an opening 61a.

The position and size of the opening 61a are set such that the opening 61a corresponds to the nasal cavity of the subject S.

A positioner 70 is installed between the shielding plate 61 and the subject S. The positioner 70 includes a contacted portion 71 and a chin rest 72.

When the subject S brings the contacted portion 71 into contact with their forehead and places their chin on the chin rest 72, the positioner 70 positions the nasal cavity of the subject S within a preset range (opening 61a). Thus, the nasal cavity of the subject S can be easily positioned.

An imager 80 is provided on the arm 11 or the hand 12 (the hand 12 in the first embodiment) to image the subject S and the specimen collection member 101. The imager 80 is a camera that captures a two-dimensional image. The imager 80 may be a camera that captures a three-dimensional image. The imager 80 images the entire face of the subject S including the nasal cavity from the front side of the subject S. The imager 80 images the specimen collection member 101 held by the hand 12. The imager 80 is arranged on a lower portion of the hand 12.

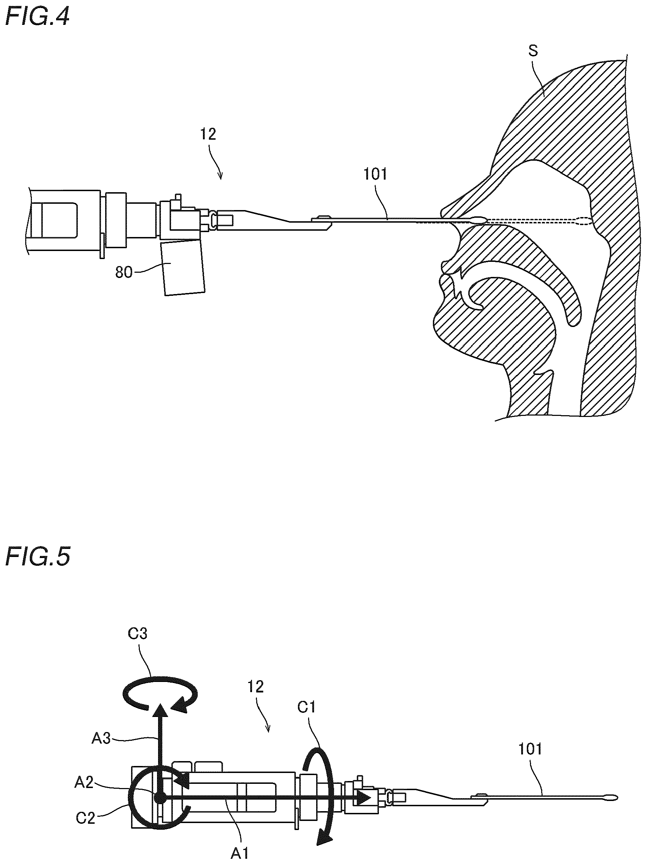

As shown in FIG. 5, the hand 12 is operated in a plurality of directions. Specifically, the hand 12 is linearly moved in a forward-rearward direction (A1 direction), a right-left direction (A2 direction), and an upward-downward direction (A3 direction), and is rotationally moved in a forward-rearward axis rotation direction (C1 direction) about a rotation axis extending in the forward-rearward direction, a right-left axis rotation direction (C2 direction) about a rotation axis extending in the right-left direction, and an upward-downward axis rotation direction (C3 direction) about a rotation axis extending in the upward-downward direction. The hand 12 is linearly moved in the forward-rearward direction, the right-left direction, and the upward-downward direction by movement of the plurality of joints 11b of the arm 11, and is rotationally moved in the forward-rearward axis rotation direction, the right-left axis rotation direction, and the upward-downward axis rotation direction. The forward-rearward direction, the right-left direction, the upward-downward direction, the forward-rearward axis rotation direction, the right-left axis rotation direction, and the upward-downward axis rotation direction are directions with respect to the hand 12. The linear movement of the hand 12 in the forward-rearward direction (A1 direction) substantially matches linear movement of the specimen collection member 101 in an axial direction. Furthermore, the rotational movement of the hand 12 in the forward-rearward axis rotation direction (C1 direction) matches rotational movement of the specimen collection member 101 about the axis.

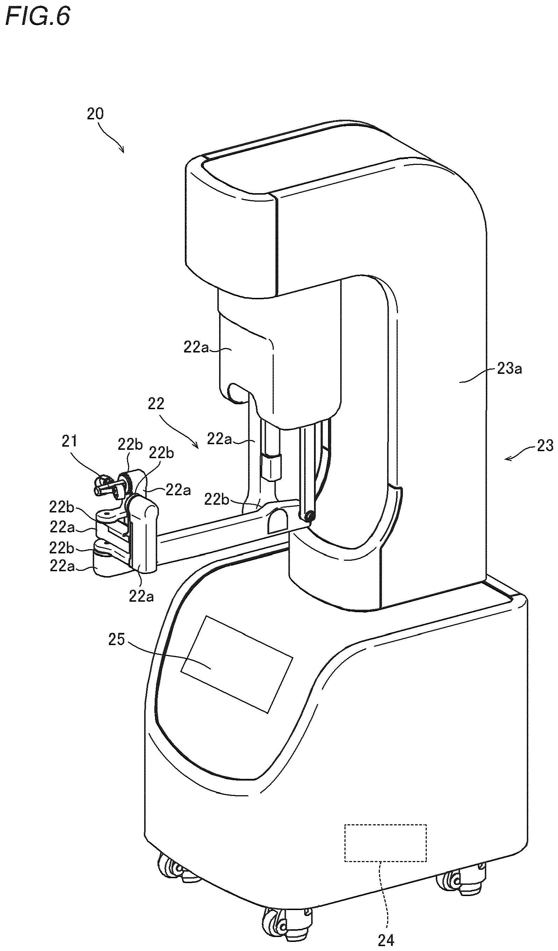

As shown in FIGS. 1 and 6, the master robot 20 remotely controls the slave robot 10. Specifically, the master robot 20 remotely controls the slave robot 10 by being operated by an operation person 0 (specimen-collecting person) such as a doctor. The master robot 20 outputs an operation command based on an operation of the operation person O. The slave robot 10 performs an action corresponding to the operation of the operation person O based on the operation command from the master robot 20.

The master robot 20 includes a movable operator 21 and an arm 22 that supports the operator 21 such that the operator 21 is movable. The operator 21 is provided to operate the slave robot 10. Specifically, the operator 21 is provided to remotely control the hand 12 of the slave robot 10 that holds the specimen collection member 101. More specifically, the operator 21 is a grip handle having a bar shape. The operator 21 receives an operation for the slave robot 10 from the operation person O moving the operator 21 while holding the operator 21 with one hand. The operator 21 is operated by the right hand of the operation person O, for example. The arm 22 includes a plurality of links 22a. The plurality of links 22a are connected to each other by a plurality of joints 22b. The plurality of joints 22b are provided. Each of the plurality of joints 22b includes a motor 121 (servomotor) (see FIG. 7) as a drive and an encoder 122 (see FIG. 7) as a position detector.

The hand 12 of the slave robot 10 moves in response to the operation of the operator 21 of the master robot 20. That is, when the operator 21 of the master robot 20 is operated in a direction corresponding to any one of the forward-rearward direction, the right-left direction, the upward-downward direction, the forward-rearward axis rotation direction, the right-left axis rotation direction, and the upward-downward axis rotation direction of the hand 12, the hand 12 of the slave robot 10 is moved in any of the forward-rearward direction, the right-left direction, the upward-downward direction, the forward-rearward axis rotation direction, the right-left axis rotation direction, and the upward-downward axis rotation direction so as to correspond to the operation of the operator 21 of the master robot 20.

The master robot 20 includes a gantry 23. The gantry 23 includes a support column 23a that bends toward the operation person O (in a substantially L-shape) on the upper side. A substantially L-shaped portion of the arm 22 is provided below the bent portion of the support column 23a.

A control unit 24 is provided in the gantry 23 to control the master robot 20. A power supply unit (not shown) etc. is provided in the gantry 23.

An operation panel 25 is provided on the outer surface of the gantry 23. The operation person O operates the operation panel 25 such that basic movement instructions, control switching, setting, etc. for the slave robot 10 and the master robot 20 are performed.

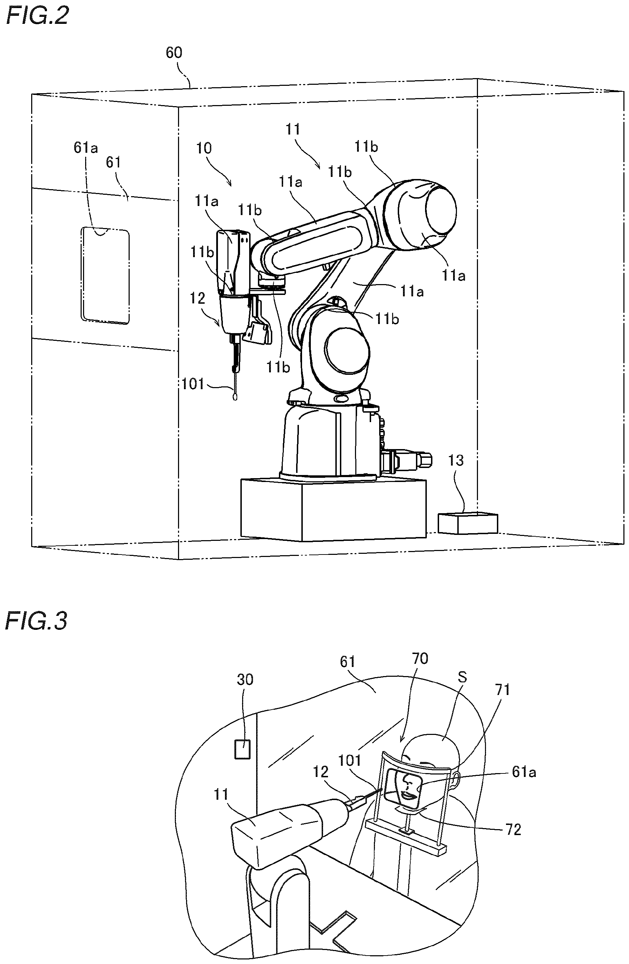

As shown in FIGS. 1 and 3, the imager 30 images the subject S and the specimen collection member 101 from the side. That is, the imager 30 images the side face of the subject S. The imager 30 is a camera that captures a two-dimensional image. The imager 30 images the entire side face of the subject S including the nose from the side of the subject S. The imager 30 images the specimen collection member 101 held by the hand 12. The imager 30 is arranged in the vicinity of the shielding plate 61 and on the subject S side of the shielding plate 61, for example.

As shown in FIG. 1, the display 40 displays an image (video) of the subject S and the specimen collection member 101. The display 40 displays an image captured by the imager 30 and an image captured by the imager 80, for example. The operation person O operates the slave robot 10 using the master robot 20 to collect a specimen from the subject S by the specimen collection member 101 while visually recognizing the real-time image of the subject S and the specimen collection member 101 displayed on the display 40. The display 40 includes a liquid crystal monitor, for example.

As shown in FIGS. 1 and 7, the controller 50 controls movement of the slave robot 10 based on an operation on the master robot 20 by the operation person O. Specifically, when the operator 21 of the master robot 20 is operated by the operation person O, the control unit 24 of the master robot 20 outputs the operation amount of the operator 21 to the controller 50 based on the detection results of the encoders 122 of the joints 22b. Then, the controller 50 calculates a movement command value for the slave robot 10 based on the operation amount of the operator 21 and outputs the calculated movement command value to the control unit 13 of the slave robot 10. Then, the control unit 13 of the slave robot 10 drives the motors 111 of the joints 11b based on the movement command value. Consequently, the hand 12 of the slave robot 10 is moved so as to correspond to the operation of the operator 21 of the master robot 20. The controller 50 includes a computer including a processor such as a CPU and memories such as a RAM and a ROM. Control software for controlling the slave robot 10 is stored in the memory of the controller 50.

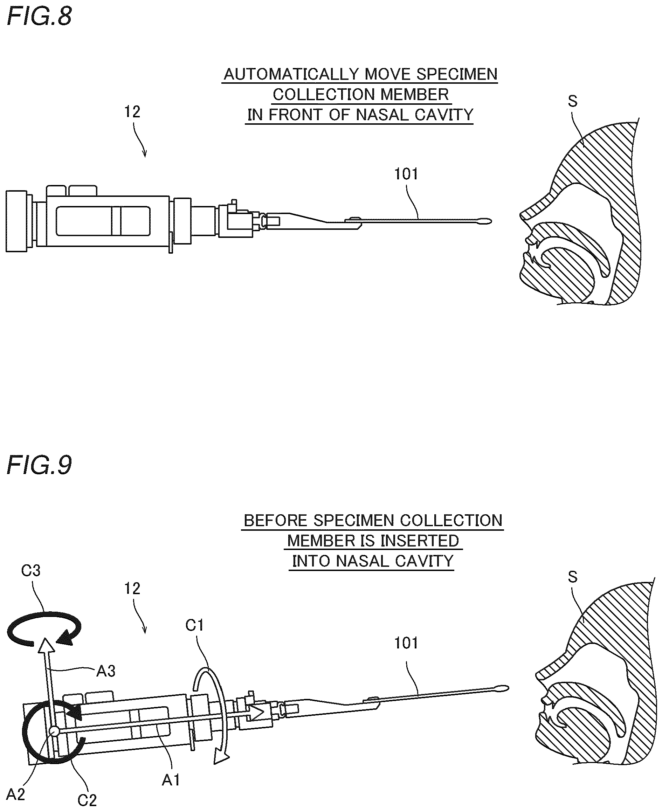

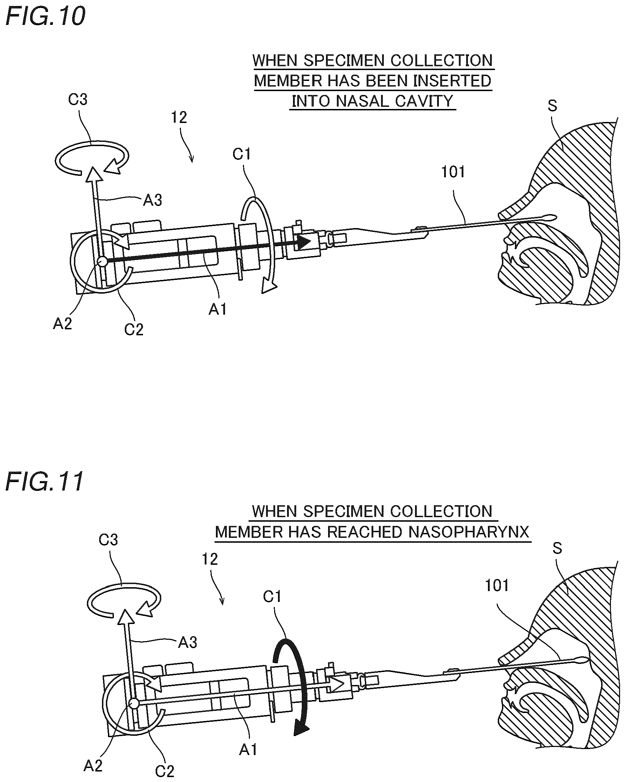

As shown in FIGS. 8 to 11, specimen collection work to collect a specimen from the subject S using the specimen collection member 101 held by the hand 12 includes a plurality of steps. Specifically, the specimen collection work includes a step of automatically placing the specimen collection member 101 in front of the nasal cavity of the subject S (automatically placing the specimen collection member 101 at an initial position) (see FIG. 8), a step of adjusting the posture of the hand 12 with respect to the nasal cavity of the subject S before inserting the specimen collection member 101 into the nasal cavity of the subject S (see FIG. 9), a step of inserting the specimen collection member 101 into the nasal cavity of the subject S (see FIG. 10), a step of rotating the specimen collection member 101 at a specimen collection position (nasopharynx) behind the nasal cavity of the subject S to collect a specimen (see FIG. 11), and a step of pulling out the specimen collection member 101 from the nasal cavity of the subject S after collecting the specimen at the specimen collection position.

When the hand 12 is freely movable in each step of the specimen collection work, there is a possibility that unnecessary movement that is not intended by the operation person O may be performed.

Therefore, in the first embodiment, the controller 50 restricts movement of the hand 12 according to the step of the specimen collection work to collect a specimen from the subject S using the specimen collection member 101 held by the hand 12.

Specifically, as shown in FIG. 9, in the first embodiment, the controller 50 restricts at least movement of the hand 12 in the forward-rearward direction (A1 direction) and enables posture change movement of the hand 12 to change the inclination of the hand 12 in a step of the specimen collection work after the specimen collection member 101 is placed in front of the nasal cavity of the subject S and before the specimen collection member 101 is inserted into the nasal cavity of the subject S. More specifically, the controller 50 restricts at least movement of the hand 12 in the forward-rearward direction (A1 direction) and enables rotational movement of the hand 12 in the right-left axis rotation direction (C2 direction) and the upward-downward axis rotation direction (C3 direction) in the step of the specimen collection work after the specimen collection member 101 is placed in front of the nasal cavity of the subject S and before the specimen collection member 101 is inserted into the nasal cavity of the subject S. At this time, when an operation is received to rotationally move the hand 12 in the right-left axis rotation direction or the upward-downward axis rotation direction, the controller 50 rotationally moves the hand 12 in the right-left axis rotation direction or the upward-downward axis rotation direction while fixing a tip end position of the specimen collection member 101.

In the first embodiment, the controller 50 restricts movement of the hand 12 other than the posture change movement of the hand 12 and enables the posture change movement of the hand 12 in the step of the specimen collection work after the specimen collection member 101 is placed in front of the nasal cavity of the subject S and before the specimen collection member 101 is inserted into the nasal cavity of the subject S. Specifically, the controller 50 restricts linear movement of the hand 12 in the forward-rearward direction (A1 direction), the right-left direction (A2 direction), and the upward-downward direction (A3 direction) and rotational movement of the hand 12 in the forward-rearward axis rotation direction (C1 direction) and enables rotational movement of the hand 12 in the right-left axis rotation direction (C2 direction) and the upward-downward axis rotation direction (C3 direction) in the step of the specimen collection work after the specimen collection member 101 is placed in front of the nasal cavity of the subject S and before the specimen collection member 101 is inserted into the nasal cavity of the subject S.

As shown in FIG. 10, in the first embodiment, the controller 50 restricts movement of the hand 12 other than movement of the hand 12 in the forward-rearward direction (A1 direction) and enables the movement of the hand 12 in the forward-rearward direction in a step of the specimen collection work at the time of inserting (during insertion of) the specimen collection member 101 into the nasal cavity of the subject S. Specifically, the controller 50 restricts linear movement of the hand 12 in the right-left direction (A2 direction) and the upward-downward direction (A3 direction) and rotational movement of the hand 12 in the forward-rearward axis rotation direction (C1 direction), the right-left axis rotation direction (C2 direction), and the upward-downward axis rotation direction (C3 direction) and enables linear movement of the hand 12 in the forward-downward direction in the step of the specimen collection work at the time of inserting the specimen collection member 101 into the nasal cavity of the subject S.

As shown in FIG. 11, in the first embodiment, the controller 50 restricts movement of the hand 12 other than rotational movement of the specimen collection member 101 of the hand 12 and enables the rotational movement of the specimen collection member 101 of the hand 12 in a step of the specimen collection work when the specimen collection member 101 has reached the specimen collection position (nasopharynx) behind the nasal cavity of the subject S. Specifically, the controller 50 restricts linear movement of the hand 12 in the forward-rearward direction (A1 direction), the right-left direction (A2 direction), and the upward-downward direction (A3 direction) and rotational movement of the hand 12 in the right-left axis rotation direction (C2 direction) and the upward-downward axis rotation direction (C3 direction) and enables rotational movement of the hand 12 in the forward-rearward axis rotation direction (C1 direction) in the step of the specimen collection work when the specimen collection member 101 has reached the specimen collection position behind the nasal cavity of the subject S. The controller 50 may restrict movement of the hand 12 other than rotational movement of the specimen collection member 101 of the hand 12 and rearward movement of the hand 12 and enable the rotational movement of the specimen collection member 101 of the hand 12 and the rearward movement of the hand 12 in the step of the specimen collection work when the specimen collection member 101 has reached the specimen collection position (nasopharynx) behind the nasal cavity of the subject S. After a specimen is collected at the specimen collection position, the hand 12 is moved rearward such that the specimen collection member 101 inside the nasal cavity of the subject S is pulled out to the outside of the nasal cavity of the subject S.

In the first embodiment, the controller 50 restricts movement of the hand 12 according to the step of the specimen collection work by controlling the master robot 20. Specifically, the controller 50 restricts the operation of the operator 21 of the master robot 20 according to the step of the specimen collection work to restrict movement of the hand 12 according to the step of the specimen collection work. More specifically, the controller 50 restricts the operation of the operator 21 of the master robot 20 by servo-locking the motor 121 (performing a control to maintain the position of the motor 121) according to the step of the specimen collection work to restrict movement of the hand 12 according to the step of the specimen collection work. In each step of the specimen collection work, the motor 121 corresponding to restriction of movement of the hand 12 among the plurality of motors 121 is servo-locked.

In the first embodiment, the controller 50 restricts movement of the hand 12 according to the step of the specimen collection work by controlling the slave robot 10. Specifically, the controller 50 restricts movement of the hand 12 according to the step of the specimen collection work by restricting the movement command value for the slave robot 10 according to the step of the specimen collection work. More specifically, the controller 50 restricts movement of the hand 12 according to the step of the specimen collection work by restricting the movement command value according to the step of the specimen collection work to restrict the operation of the motor 111 based on the movement command value. In each step of the specimen collection work, the plurality of motors 111 are not operated according to restriction of movement of the hand 12.

In the first embodiment, the controller 50 restricts movement of the hand 12 according to the step of the specimen collection work by controlling both the slave robot 10 and the master robot 20.

In the first embodiment, the controller 50 detects the step of the specimen collection work based on an operation on the master robot 20 by the operation person O. Specifically, the controller 50 detects the step of the specimen collection work based on an operation on the operation panel 25 by the operation person O. When the step of the specimen collection work is switched, the operation person O performs a step switching operation using the operation panel 25. The controller 50 detects the step of the specimen collection work based on the step switching operation by the operation person O using the operation panel 25.

In the first embodiment, the controller 50 detects the step of the specimen collection work based on at least one of the image (video) of the subject S and the specimen collection member 101 captured by the imager 30 or position information (coordinate information) on the slave robot 10. Specifically, the controller 50 detects the step of the specimen collection work based on both the image of the subject S and the specimen collection member 101 captured by the imager 30 and the position information on the slave robot 10. More specifically, the controller 50 detects the step of the specimen collection work by detecting the insertion state of the specimen collection member 101 into the nasal cavity of the subject S based on the recognition result of the image of the subject S and the specimen collection member 101 captured by the imager 30 and the position information on the hand 12 of the slave robot 10. The position information on the hand 12 can be acquired based on outputs of the encoders 112 of the plurality of joints 11b.

Only one or both of detection of the step of the specimen collection work based on an operation on the master robot 20 by the operation person O and detection of the step of the specimen collection work based on the image (video) of the subject S and the specimen collection member 101 captured by the imager 30 may be performed.

### Control Process for Specimen Collection Work

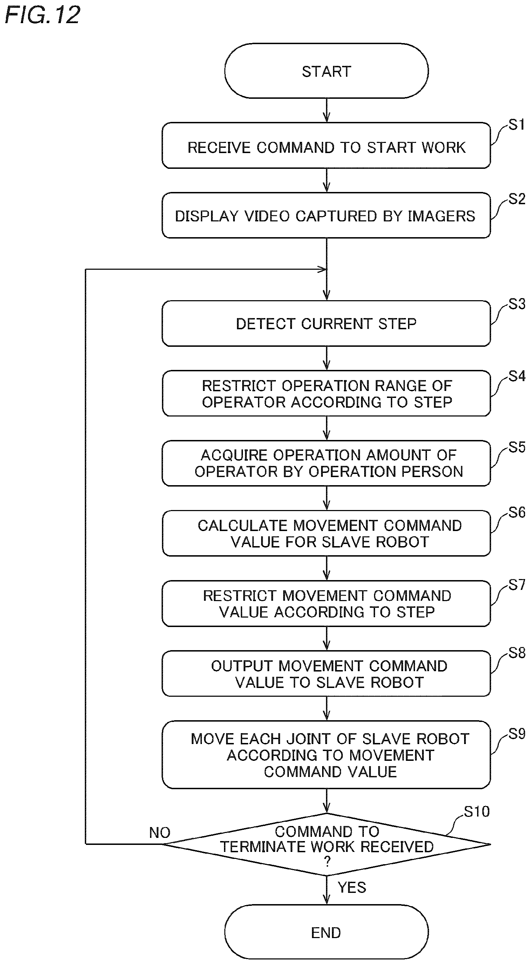

A control process for the specimen collection work of the specimen collection robot system 100 according to the first embodiment is now described based on a flowchart with reference to FIG. 12.

As shown in FIG. 12, first, in step S1, a command to start the specimen collection work is received.

Then, in step S2, images (video) captured by the imager 30 and the imager 80 are displayed on the display 40.

Then, in step S3, the current step of the specimen collection work is detected. In step S3, the current step of the specimen collection work is detected based on an operation on the operation panel 25 by the operation person O, or at least one of the image of the subject S and the specimen collection member 101 captured by the imager 30 or the position information (coordinate information) on the slave robot 10.

Then, in step S4, the operation range of the operator 21 of the master robot 20 is restricted by the servo locking of the motor 121 according to the step of the specimen collection work detected in step S3.

Then, in step S5, the operation amount of the operator 21 by the operation person O is acquired.

Then, in step S6, the movement command value for the slave robot 10 is calculated based on the operation amount of the operator 21 by the operation person O acquired in

### step S5.

Then, in step S7, the movement command value for the slave robot 10 is restricted according to the step of the specimen collection work detected in step S3.

Then, in step S8, the movement command value restricted in step S7 is output to the slave robot 10.

Then, in step S9, each joint 11b of the slave robot 10 is moved based on the movement command value output in step S8. Thus, the hand 12 of the slave robot 10 is moved in any one of the forward-rearward direction, the right-left direction, the upward-downward direction, the forward-rearward axis rotation direction, the right-left axis rotation direction, and the upward-downward axis rotation direction.

Then, in step S10, it is determined whether or not a command to terminate the specimen collection work has been received. When it is determined that the command to terminate the specimen collection work has not been received, the process returns to step S3. Then, the process operations in step S3 to step S9 are repeated. When it is determined that the command to terminate the specimen collection work has been received, the control process for the specimen collection work is terminated.

### Advantages of First Embodiment

According to the first embodiment, the following advantages are achieved.

According to the first embodiment, as described above, the specimen collection robot system 100 includes the controller 50 configured or programmed to restrict movement of the hand 12 according to the step of the specimen collection work to collect a specimen from the subject S using the specimen collection member 101 held by the hand 12. Accordingly, movement of the hand 12 is appropriately restricted according to the step of the specimen collection work, and thus unlike a case in which no restriction is set on movement of the hand 12, unintended and unnecessary movement of the hand 12 can be reduced or prevented. Consequently, movement of the hand 12 suitable for the step of the specimen collection work can be performed, and thus even when the master robot 20 remotely controls the hand 12 of the slave robot 10 to perform a procedure (specimen collection work) on the subject S, the procedure (specimen collection work) on the subject S can be easily performed. Thus, the workability of the procedure (specimen collection work) on the subject S can be improved.

According to the first embodiment, as described above, the controller 50 is configured or programmed to restrict at least movement of the hand 12 in the forward-rearward direction and enable the posture change movement of the hand 12 to change the inclination of the hand 12 in the step of the specimen collection work after the specimen collection member 101 is placed in front of the nasal cavity of the subject S and before the specimen collection member 101 is inserted into the nasal cavity of the subject S. Accordingly, when the posture of the hand 12 is changed in order to insert the specimen collection member 101 into the nasal cavity of the subject S in the step of the specimen collection work after the specimen collection member 101 is placed in front of the nasal cavity of the subject S and before the specimen collection member 101 is inserted into the nasal cavity of the subject S, the necessary posture change movement of the hand 12 can be performed while unnecessary movement of the hand 12 in the forward-rearward direction is reduced or prevented. Consequently, the work before insertion of the specimen collection member 101 into the nasal cavity of the subject S (work to adjust the posture of the hand 12) can be easily performed, and thus the workability of adjusting the posture of the hand 12 before insertion of the specimen collection member 101 into the nasal cavity of the subject S can be improved.

According to the first embodiment, as described above, the controller 50 is configured or programmed to restrict movement of the hand 12 other than the posture change movement of the hand 12 and enable the posture change movement of the hand 12 in the step of the specimen collection work after the specimen collection member 101 is placed in front of the nasal cavity of the subject S and before the specimen collection member 101 is inserted into the nasal cavity of the subject S. Accordingly, when the posture of the hand 12 is changed in order to insert the specimen collection member 101 into the nasal cavity of the subject S in the step of the specimen collection work after the specimen collection member 101 is placed in front of the nasal cavity of the subject S and before the specimen collection member 101 is inserted into the nasal cavity of the subject S, the necessary posture change movement of the hand 12 can be performed while unnecessary movement of the hand 12 other than the posture change movement is reduced or prevented. Consequently, the work before insertion of the specimen collection member 101 into the nasal cavity of the subject S (work to adjust the posture of the hand 12) can be more easily performed, and thus the workability of adjusting the posture of the hand 12 before insertion of the specimen collection member 101 into the nasal cavity of the subject S can be further improved.

According to the first embodiment, as described above, the controller 50 is configured or programmed to restrict movement of the hand 12 other than movement of the hand 12 in the forward-rearward direction and enable the movement of the hand 12 in the forward-rearward direction in the step of the specimen collection work at the time of inserting the specimen collection member 101 into the nasal cavity of the subject S. Accordingly, the necessary movement of the hand 12 in the forward-rearward direction can be performed while the unnecessary movement of the hand 12 (such as the posture change movement) other than the movement of the hand 12 in the forward-rearward direction is reduced or prevented during insertion of the specimen collection member 101 into the nasal cavity of the subject S in the step of the specimen collection work at the time of inserting the specimen collection member 101 into the nasal cavity of the subject S. Consequently, the work to insert the specimen collection member 101 into the nasal cavity of the subject S (work to move the specimen collection member 101 forward or rearward within the nasal cavity using the hand 12) can be easily performed, and thus the workability of inserting the specimen collection member 101 into the nasal cavity of the subject S can be improved.

According to the first embodiment, as described above, the controller 50 is configured or programmed to, in the step of the specimen collection work when the specimen collection member 101 has reached the specimen collection position behind the nasal cavity of the subject S, restrict movement of the hand 12 other than rotational movement of the specimen collection member 101 of the hand 12 and enable the rotational movement of the specimen collection member 101 of the hand 12, or restrict movement of the hand 12 other than the rotational movement of the specimen collection member 101 of the hand 12 and rearward movement of the hand 12 and enable the rotational movement of the specimen collection member 101 of the hand 12 and the rearward movement of the hand 12. Accordingly, when the movement of the hand 12 other than the rotational movement of the specimen collection member 101 of the hand 12 is restricted, and the rotational movement of the specimen collection member 101 of the hand 12 is enabled, the necessary rotational movement of the specimen collection member 101 of the hand 12, or the necessary rotational movement of the specimen collection member 101 of the hand 12 and the necessary rearward movement of the hand 12 can be performed while the unnecessary movement of the hand 12 other than the rotational movement of the specimen collection member 101 of the hand 12, or the unnecessary movement of the hand 12 other than the rotational movement of the specimen collection member 101 of the hand 12 and the rearward movement of the hand 12 is reduced or prevented when the specimen collection member 101 is rotated in order to collect a specimen at the specimen collection position behind the nasal cavity of the subject S in the step of the specimen collection work when the specimen collection member 101 has reached the specimen collection position behind the nasal cavity of the subject S. Consequently, the work to collect a specimen at the specimen collection position behind the nasal cavity of the subject S using the specimen collection member 101 (work to rotate the specimen collection member 101 at the specimen collection position using the hand 12) can be easily performed, and thus the workability of the specimen collection work at the specimen collection position can be improved.

According to the first embodiment, as described above, the master robot 20 includes the movable operator 21 to operate the slave robot 10. The hand 12 is operable to move in response to the operation of the operator 21. The controller 50 is configured or programmed to restrict movement of the hand 12 according to the step of the specimen collection work by restricting the operation of the operator 21 of the master robot 20 according to the step of the specimen collection work. Accordingly, even when an attempt is made to operate the operator 21 of the master robot 20 such that unnecessary movement of the hand 12 is performed, the operation of the operator 21 of the master robot 20 is restricted, and thus unintended and unnecessary movement of the hand 12 can be easily reduced or prevented.

According to the first embodiment, as described above, the controller 50 is configured or programmed to restrict movement of the hand 12 according to the step of the specimen collection work by restricting the movement command value for the slave robot 10 according to the step of the specimen collection work. Accordingly, even when the master robot 20 is operated such that unnecessary movement of the hand 12 is performed, the unnecessary movement of the hand 12 is not reflected in the movement command value for the slave robot 10, and thus unintended and unnecessary movement of the hand 12 can be easily reduced or prevented.

According to the first embodiment, as described above, the slave robot 10 includes a vertical articulated robot having the plurality of joints 11b. Each of the plurality of joints 11b includes the motor 111. The controller 50 is configured or programmed to restrict movement of the hand 12 according to the step of the specimen collection work by restricting the movement command value according to the step of the specimen collection work to restrict the operation of the motor 111 based on the movement command value. Accordingly, the operation of the motor 111 of the slave robot 10 (vertical articulated robot) that would cause unnecessary movement of the hand 12 is not performed, and thus unintended and unnecessary movement of the hand 12 can be more easily reduced or prevented.

According to the first embodiment, as described above, the controller 50 is configured or programmed to detect the step of the specimen collection work based on an operation on the master robot 20 by the operation person O. Accordingly, the step of the specimen collection work can be accurately detected based on the operation (determination) on the master robot 20 by the operation person O, and thus movement of the hand 12 can be restricted according to the step of the specimen collection work at the accurate timing.

According to the first embodiment, as described above, the controller 50 is configured or programmed to detect the step of the specimen collection work based on at least one of the image of the subject S and the specimen collection member 101 captured by the imager 30 or the position information on the slave robot 10. Accordingly, the step of the specimen collection work can be automatically detected based on at least one of the image of the subject S and the specimen collection member 101 captured by the imager 30 or the position information on the slave robot 10, and thus time and effort in determining the step of the specimen collection work can be saved.

### Second Embodiment

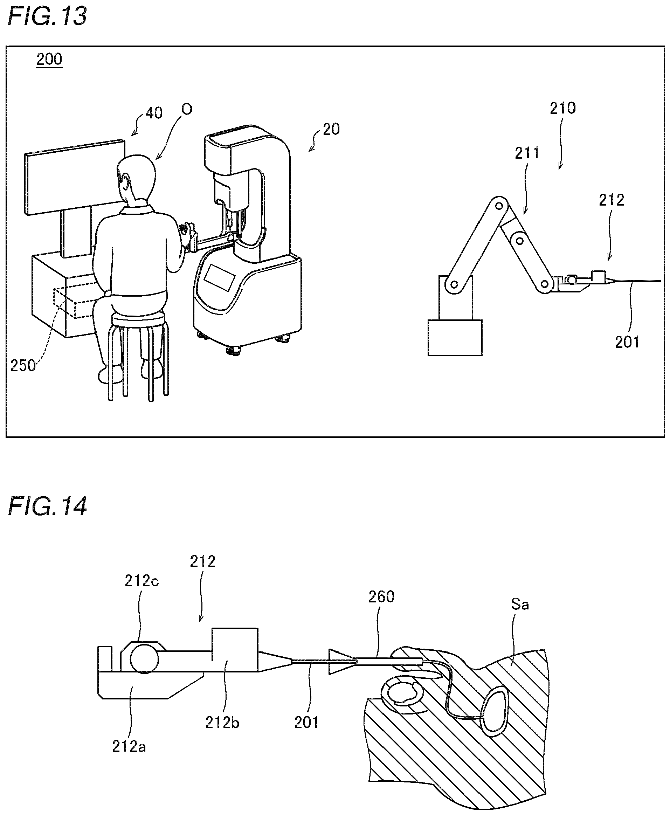

The configuration of a robot system 200 according to a second embodiment is now described with reference to FIGS. 13 to 18. The robot system 200 performs ureteroscopic surgery, unlike the first embodiment in which a specimen is collected. The same or similar configurations as those of the first embodiment are denoted by the same reference numerals, and detailed description thereof is omitted.

As shown in FIG. 13, the robot system 200 according to the second embodiment includes a slave robot 210, a master robot 20, an imager 30, a display 40, and a controller 250.

As shown in FIGS. 13 and 14, the slave robot 210 performs a procedure to perform ureteroscopic surgery on a person to be treated Sa (patient) to crush a urinary calculus using a treatment member 201. The treatment member 201 is a bendable flexible tube. The slave robot 210 inserts the treatment member 201 into the urethra of the person to be treated Sa through a ureteral access sheath 260 inserted in advance into the urethra of the person to be treated Sa by a medical staff, and images the inside of the urinary tract of the person to be treated Sa, such as the urethra and ureter. The captured video is displayed on the display 40. An operation person O operates the slave robot 210 using the master robot 20 to perform ureteroscopic surgery on the person to be treated Sa while checking the real-time video of the person to be treated Sa displayed on the display 40. In the ureteroscopic surgery, a calculus site of the person to be treated Sa is identified, the calculus is crushed by radiating laser light with a laser forceps, and the calculus is retrieved with a basket forceps, for example.

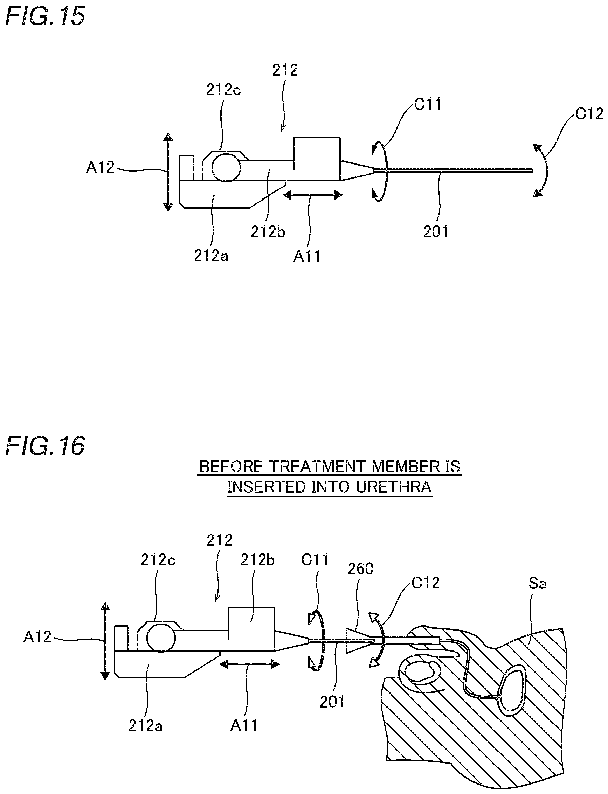

The slave robot 210 is a vertical articulated robot. The slave robot 210 includes an arm 211 and a hand 212 attached to the tip end of the arm 211. The arm 211 has a plurality of joints. Each of the plurality of joints of the arm 211 includes a drive such as a servomotor and a position detector such as an encoder. The hand 212 holds the treatment member 201, which is a bendable flexible tube. The hand 212 includes a hand base 212a, a ureteroscope main body 212b, and a motor 212c. The hand base 212a is attached to the tip end of the arm 211. The ureteroscope main body 212b can image the inside of the urinary tract of the person to be treated Sa. A laser forceps for crushing a urinary calculus of the person to be treated Sa or a basket forceps for retrieving the urinary calculus can be inserted into the ureteroscope main body 212b. The ureteroscope main body 212b holds the treatment member 201. The motor 212c rotates the ureteroscope main body 212b in a C12 direction (see FIG. 15) described below.

As shown in FIG. 15, the hand 212 is operated in a plurality of directions. Specifically, the hand 212 is moved straight ahead in a forward-rearward direction (All direction) and in an upward-downward direction (A12 direction), and is rotationally moved in a first rotation direction (C11 direction) about a first rotation axis extending in the forward-rearward direction and in a second rotation direction (C12 direction) about a second rotation axis extending in a right-left direction. The forward-rearward direction, the right-left direction, the upward-downward direction, the first rotation direction, and the second rotation direction are directions with respect to the hand 212. When the treatment member 201 is inserted into the urethra of the person to be treated Sa, the hand 212 is moved straight ahead in a forward direction (insertion direction).

As shown in FIG. 13, the controller 250 controls movement of the slave robot 210 based on an operation on the master robot 20 by the operation person O. Specifically, when an operator 21 of the master robot 20 is operated by the operation person O, a control unit 24 of the master robot 20 outputs the operation amount of the operator 21 to the controller 250 based on the detection results of encoders 122 of joints 22b. Then, the controller 250 calculates a movement command value for the slave robot 210 based on the operation amount of the operator 21, and outputs the calculated movement command value to a control unit of the slave robot 210. Then, the control unit of the slave robot 210 drives motors of the joints and the motor 212c of the hand 212 based on the movement command value. Consequently, the hand 212 of the slave robot 210 is moved so as to correspond to the operation of the operator 21 of the master robot 20. The controller 250 includes a computer including a processor such as a CPU and memories such as a RAM and a ROM. Control software for controlling the slave robot 210 is stored in the memory of the controller 250.

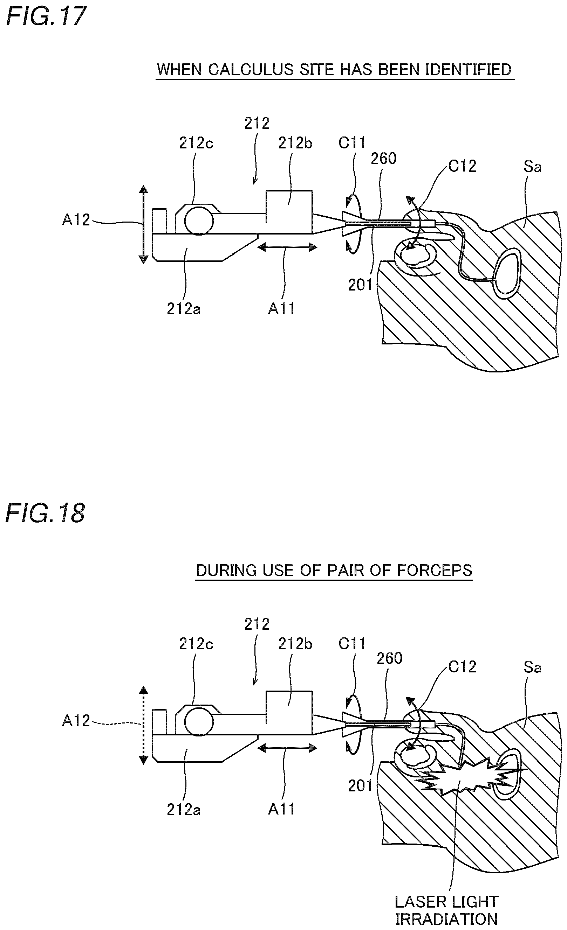

As shown in FIGS. 15 to 18, the procedure to perform ureteroscopic surgery on the person to be treated Sa using the treatment member 201 held by the hand 212 includes a plurality of steps. Specifically, the procedure to perform ureteroscopic surgery includes a step of inserting the treatment member 201 into the urethra of the person to be treated Sa through the ureteral access sheath 260 (see FIG. 16), a step in which the operation person O identifies the calculus site of the person to be treated Sa while checking the video displayed on the display 40 after the treatment member 201 is inserted into the urethra of the person to be treated Sa (see FIG. 17), and a step in which the operation person O uses a forceps via the ureteroscope main body 212b and the treatment member 201 for the calculus of the person to be treated Sa (see FIG. 18).

In the second embodiment, the controller 50 restricts movement of the hand 212 according to the step of the procedure performed on the person to be treated Sa using the treatment member 201 held by the hand 212.

Specifically, as shown in FIG. 16, in the second embodiment, the controller 250 restricts movement of the hand 212 other than movement of the hand 212 in the forward-rearward direction (All direction) and the upward-downward direction (A12 direction) and enables the movement of the hand 212 in the forward-rearward direction and the upward-downward direction in the step of the procedure before the treatment member 201 is inserted into the urethra of the person to be treated Sa. More specifically, the controller 250 restricts rotational movement of the hand 212 in the first rotation direction (C11) and the second rotation direction (C12) and enables movement of the hand 212 in the forward-rearward direction and the upward-downward direction in the step of the procedure before the treatment member 201 is inserted into the urethra of the person to be treated Sa.

As shown in FIG. 17, in the second embodiment, the controller 250 does not restrict but enables movement of the hand 212 in the forward-rearward direction (All direction), movement of the hand 212 in the upward-downward direction (A12 direction), rotational movement of the treatment member 201 of the hand 212, and posture change movement of the hand 212 to change the inclination of the hand 212 in the step of the procedure after the treatment member 201 is inserted into the urethra of the person to be treated Sa and before the calculus site of the person to be treated Sa is identified. Specifically, the controller 250 does not restrict but enables movement of the hand 212 in the forward-rearward direction (All direction), movement of the hand 212 in the upward-downward direction (A12 direction), rotational movement of the hand 212 in the first rotation direction (C11 direction), and rotational movement of the hand 212 in the second rotation direction (C12 direction) in the step of the procedure after the treatment member 201 is inserted into the urethra of the person to be treated Sa and before the calculus site of the person to be treated Sa is identified.

As shown in FIG. 18, in the second embodiment, the controller 250 does not restrict but enables movement of the hand 212 in the forward-rearward direction (All direction), rotational movement of the treatment member 201 of the hand 212, and the posture change movement to change the inclination of the hand 212, and automatically moves the hand 212 in the upward-downward direction (A12 direction) in the step of the procedure during use of the forceps (the laser forceps or the basket forceps) for the calculus of the person to be treated Sa. Specifically, the controller 250 does not restrict but enables movement of the hand 212 in the forward-rearward direction (All direction), rotational movement of the hand 212 in the first rotation direction (C11 direction), and rotational movement of the hand 212 in the second rotation direction (C12 direction), and automatically moves the hand 212 in the upward-downward direction (A12 direction) in the step of the procedure during use of the forceps for the calculus of the person to be treated Sa.

The controller 250 automatically moves the hand 212 in the upward-downward direction (A12 direction) such that the hand 212 moves up and down in accordance with the breathing motion of the person to be treated Sa under anesthesia. The controller 250 acquires parameters (breathing cycle, ventilation volume, etc.) related to the breathing of the person to be treated Sa from an anesthesia apparatus, for example, and moves the hand 212 up and down in accordance with the breathing motion of the person to be treated Sa based on the acquired parameters.

The remaining configurations of the second embodiment are similar to those of the first embodiment.

### Advantages of Second Embodiment

According to the second embodiment, the following advantages are achieved.

According to the second embodiment, as described above, the robot system 200 includes the controller 250 configured or programmed to restrict movement of the hand 212 according to the step of the procedure on the person to be treated Sa using the treatment member 201 held by the hand 212. Accordingly, similarly to the first embodiment, even when the hand 212 of the slave robot 210 is remotely controlled by the master robot 20 to perform the procedure on the person to be treated Sa, the procedure can be easily performed on the person to be treated Sa.

According to the second embodiment, as described above, the controller 250 is configured or programmed to restrict movement of the hand 212 other than movement of the hand 212 in the forward-rearward direction and the upward-downward direction and enable the movement of the hand 212 in the forward-rearward direction and the upward-downward direction in the step of the procedure before the treatment member 201 is inserted into the urethra of the person to be treated Sa. Accordingly, in the step of the procedure before the treatment member 201 is inserted into the urethra of the person to be treated Sa, the necessary movement of the hand 212 in the forward-rearward direction and the upward-downward direction can be performed while the unnecessary movement of the hand 212 (such as the posture change movement) other than the movement of the hand 212 in the forward-rearward direction and the upward-downward direction is reduced or prevented during insertion of the treatment member 201 into the urethra of the person to be treated Sa. Consequently, the treatment member 201 can be easily inserted into the urethra of the person to be treated Sa, and thus the workability of inserting the treatment member 201 into the urethra of the person to be treated Sa can be improved.

According to the second embodiment, as described above, the controller 250 is configured or programmed to not restrict but to enable movement of the hand 212 in the forward-rearward direction, movement of the hand 212 in the upward-downward direction, rotational movement of the treatment member 201 of the hand 212, and the posture change movement of the hand 212 to change the inclination of the hand 212 in the step of the procedure after the treatment member 201 is inserted into the urethra of the person to be treated Sa and before the calculus site of the person to be treated Sa is identified. Accordingly, in the step of the procedure after the treatment member 201 is inserted into the urethra of the person to be treated Sa and before the calculus site of the person to be treated Sa is identified, the necessary movement of the hand 212 in the forward-rearward direction, the necessary movement of the hand 212 in the upward-downward direction, the necessary rotational movement of the treatment member 201 of the hand 212, and the necessary posture change movement can be performed. Consequently, the calculus of the person to be treated Sa can be easily identified, and thus the workability of identifying the calculus of the person to be treated Sa can be improved.

According to the second embodiment, as described above, the controller 250 is configured or programmed to not restrict but to enable movement of the hand 212 in the forward-rearward direction, rotational movement of the treatment member 201 of the hand 212, and the posture change movement of the hand 212 to change the inclination of the hand 212, and automatically move the hand 212 in the upward-downward direction in the step of the procedure during use of the forceps for the calculus of the person to be treated Sa. Accordingly, in the step of the procedure during use of the forceps for the calculus of the person to be treated Sa, the necessary movement of the hand 212 in the forward-rearward direction, the necessary rotational movement of the treatment member 201 of the hand 212, and the necessary posture change movement can be performed. Furthermore, the hand 212 can be automatically moved in the upward-downward direction in accordance with the breathing motion of the person to be treated Sa. Consequently, the work using the forceps for the calculus of the person to be treated Sa can be easily performed, and thus the workability of the work using the forceps for the calculus of the person to be treated Sa can be improved.

The remaining advantages of the second embodiment are similar to those of the first embodiment.

### Modified Examples

The embodiments disclosed this time must be considered as illustrative in all points and not restrictive. The scope of the present disclosure is not shown by the above description of the embodiments but by the scope of claims for patent, and all modifications (modified examples) within the meaning and scope equivalent to the scope of claims for patent are further included.

For example, while the example in the slave robot is a vertical articulated robot has been shown in each of the aforementioned first and second embodiments, the present disclosure is not limited to this. For example, the slave robot may be a robot such as a horizontal articulated robot or a dual-arm robot.

While the example in which the movement of the hand other than the posture change movement of the hand is restricted, and the posture change movement of the hand is enabled in the step of the specimen collection work after the specimen collection member is placed in front of the nasal cavity of the subject and before the specimen collection member is inserted into the nasal cavity of the subject has been shown in the aforementioned first embodiment, the present disclosure is not limited to this. In the present disclosure, it is not necessary to restrict all movement of the hand other than the posture change movement of the hand as long as the posture change movement of the hand is enabled in the step of the specimen collection work after the specimen collection member is placed in front of the nasal cavity of the subject and before the specimen collection member is inserted into the nasal cavity of the subject. For example, the movement of the hand in the forward-rearward direction may be restricted, and the movement of the hand in the right-left direction and the upward-downward direction, the rotational movement of the specimen collection member of the hand, and the posture change movement of the hand may be enabled. Alternatively, the movement of the hand in the forward-rearward direction and the rotational movement of the specimen collection member of the hand may be restricted, and the movement of the hand in the right-left direction and the upward-downward direction, and the posture change movement of the hand may be enabled, for example.

While the example in which the movement of the hand other than the movement of the hand in the forward-rearward direction is restricted, and the movement of the hand in the forward-rearward direction is enabled in the step of the specimen collection work at the time of inserting the specimen collection member into the nasal cavity of the subject has been shown in the aforementioned first embodiment, the present disclosure is not limited to this. In the present disclosure, it is not necessary to restrict all movement of the hand other than the movement of the hand in the forward-rearward direction as long as the movement of the hand in the forward-rearward direction is enabled in the step of the specimen collection work at the time of inserting the specimen collection member into the nasal cavity of the subject. For example, the movement of the hand in the right-left direction and the upward-downward direction and the posture change movement of the hand may be restricted, and the rotational movement of the specimen collection member of the hand and the movement of the hand in the forward-rearward direction may be enabled.

While the example in which the movement of the hand is restricted according to the step of the specimen collection work by restricting both the operation of the operator of the master robot and the movement command value for the slave robot has been shown in each of the aforementioned first and second embodiments, the present disclosure is not limited to this. For example, the movement of the hand may be restricted according to the step of the specimen collection work by restricting only one of the operation of the operator of the master robot and the movement command value for the slave robot.

While the example in which the step of the procedure is detected based on both the image of the subject and the specimen collection member captured by the imager and the position information on the slave robot has been shown in each of the aforementioned first and second embodiments, the present disclosure is not limited to this. For example, the step of the procedure may be detected based on only one of the image of the subject and the specimen collection member captured by the imager and the position information on the slave robot.

While the example in which the robot system is a robot system that collects a specimen from a subject using the specimen collection member has been shown in the aforementioned first embodiment, and the example in which the robot system is a robot system that performs ureteroscopic surgery on a person to be treated using the treatment member has been shown in the aforementioned second embodiment, the present disclosure is not limited to this. In the present disclosure, the robot system may be a robot system that performs a procedure other than specimen collection and ureteroscopic surgery on a subject (person to be treated). For example, the robot system may be a robot system that examines a subject (person to be treated) using a medical inspection instrument (treatment member).

### Description of Reference Numerals

10, 210: slave robot
11b: joint
111: motor
12, 212: hand
20: master robot
21: operator
30: imager
50, 250: controller
100: specimen collection robot system (robot system)
101: specimen collection member (treatment member)
200: robot system
201: treatment member
O: operation person
S: subject (person to be treated)
Sa: person to be treated

## Claims

1. A robot system comprising:
a slave robot including a hand to hold a treatment member and operable to perform a procedure on a person to be treated using the treatment member held by the hand;
a master robot to remotely control the slave robot; and
a controller configured or programmed to restrict movement of the hand according to a step of the procedure on the person to be treated using the treatment member held by the hand.

2. The robot system according to claim 1,
wherein
the treatment member includes a member to be inserted into a urethra of the person to be treated; and
the controller is configured or programmed to restrict movement of the hand other than movement of the hand in a forward-rearward direction and in an upward-downward direction and enable the movement of the hand in the forward-rearward direction and in the upward-downward direction in a step of the procedure before the treatment member is inserted into the urethra of the person to be treated.

3. The robot system according to claim 1,
wherein
the treatment member includes a member to be inserted into a urethra of the person to be treated; and
the controller is configured or programmed to not restrict but to enable movement of the hand in a forward-rearward direction, movement of the hand in an upward-downward direction, rotational movement of the treatment member of the hand, and posture change movement of the hand to change an inclination of the hand in a step of the procedure after the treatment member is inserted into the urethra of the person to be treated and before a calculus site of the person to be treated is identified.

4. The robot system according to claim 1,
wherein
the treatment member includes a member to be inserted into a urethra of the person to be treated; and
the controller is configured or programmed to not restrict but to enable movement of the hand in a forward-rearward direction, rotational movement of the treatment member of the hand, and posture change movement of the hand to change an inclination of the hand, and automatically move the hand in an upward-downward direction in a step of the procedure during use of a forceps for a calculus of the person to be treated.

5. The robot system according to claim 1,
wherein
the treatment member includes a member to be inserted into a nasal cavity of the person to be treated; and
the controller is configured or programmed to restrict at least movement of the hand in a forward-rearward direction and enable posture change movement of the hand to change an inclination of the hand in a step of the procedure after the treatment member is placed in front of the nasal cavity of the person to be treated and before the treatment member is inserted into the nasal cavity of the person to be treated.

6. The robot system according to claim 5,
wherein the controller is configured or programmed to restrict movement of the hand other than the posture change movement of the hand and enable the posture change movement of the hand in the step of the procedure after the treatment member is placed in front of the nasal cavity of the person to be treated and before the treatment member is inserted into the nasal cavity of the person to be treated.

7. The robot system according to claim 1,
wherein
the treatment member includes a member to be inserted into a nasal cavity of the person to be treated; and
the controller is configured or programmed to restrict movement of the hand other than movement of the hand in a forward-rearward direction and enable the movement of the hand in the forward-rearward direction in a step of the procedure at a time of inserting the treatment member into the nasal cavity of the person to be treated.

8. The robot system according to claim 1,
wherein
the treatment member includes a member to be inserted into a nasal cavity of the person to be treated; and
the controller is configured or programmed to, in a step of the procedure when the treatment member has reached a procedure position behind the nasal cavity of the person to be treated, restrict movement of the hand other than rotational movement of the treatment member of the hand and enable the rotational movement of the treatment member of the hand, or restrict movement of the hand other than the rotational movement of the treatment member of the hand and rearward movement of the hand and enable the rotational movement of the treatment member of the hand and the rearward movement of the hand.

9. The robot system according to claim 1,
wherein
the master robot includes a movable operator to operate the slave robot;
the hand is operable to move in response to operation of the operator; and
the controller is configured or programmed to restrict movement of the hand according to the step of the procedure by restricting the operation of the operator of the master robot according to the step of the procedure.

10. The robot system according to claim 1,
wherein the controller is configured or programmed to restrict movement of the hand according to the step of the procedure by restricting a movement command value for the slave robot according to the step of the procedure.

11. The robot system according to claim 10,
wherein
the slave robot includes a vertical articulated robot having a plurality of joints;
each of the plurality of joints includes a motor; and
the controller is configured or programmed to restrict movement of the hand according to the step of the procedure by restricting the movement command value according to the step of the procedure to restrict operation of the motor based on the movement command value.

12. The robot system according to claim 1,
wherein the controller is configured or programmed to detect the step of the procedure based on an operation on the master robot by an operation person.

13. The robot system according to claim 1,
wherein the controller is configured or programmed to detect the step of the procedure based on at least one of an image of the person to be treated and the treatment member captured by an imager or a position information on the slave robot.
